# EUROPÄISCHE PATENTSCHRIFT

(11) **EP 1 611 473 B1**
(45) Veröffentlichungstag und Bekanntmachung des Hinweises auf die Patenterteilung: **09.04.2008**
(21) Anmeldenummer: 04721849.0
(22) Anmeldetag: 19.03.2004
(51) Int. Cl.: G02B 27/00, A61B 3/12, G02B 13/08

(54) **OPTISCHES SYSTEM FÜR EINE FUNDUSKAMERA**
OPTICAL SYSTEM FOR A FUNDUS CAMERA
SYSTEME OPTIQUE POUR CAMERA A FOND D'OEIL

(30) Priorität: 10.04.2003 DE 10316416
(43) Veröffentlichungstag der Anmeldung: 04.01.2006
(73) Patentinhaber: Carl Zeiss Meditec AG, 07745 Jena (DE)
(72) Erfinder: MUELLER, Lothar, 07646 Ottendorf (DE); HANFT, Marco, 07743 Jena (DE); LIPPMANN, Uwe, 07745 Jena (DE)
(74) Vertreter: Beck, Bernard
(86) Internationale Anmeldenummer: PCT/EP2004/002893
(87) Internationale Veröffentlichungsnummer: WO 2004/090606

(56) Entgegenhaltungen:
- US-A- 4 415 239
- US-A- 4 730 910
- US-A- 5 640 275

## Beschreibung

Die Erfindung betrifft ein optisches System für eine Funduskamera, welche zur Abbildung des Augenhintergrundes dient. Bei der Abbildung des Augenhintergrundes mit einer solchen Kamera kommt es im allgemeinen zu Reflexen an der Hornhaut und an Flächen des optischen Systems, welche sich störend auf die Bildqualität auswirken.

Der grundsätzliche Aufbau einer Funduskamera weist ein mehrstufiges optisches System auf. Eine Ophthalmoskoplinse erzeugt ein Zwischenbild, das von einem Folgesystem (Hauptobjektiv) auf einen Film oder eine CCD-Matrix abgebildet wird. Die Ophthalmoskoplinse ist auch Bestandteil der Beleuchtung.
Ein besonderes Problem bei der Fundusbeobachtung und -aufnahme stellen Reflexe an der Hornhaut und den Flächen der Ophthalmoskoplinse dar, weil das von der Netzhaut reflektierte Licht, welches die eigentlich interessierende Bildinformation trägt, wesentlich weniger intensiv ist als das vor dem Eintritt in das Auge reflektierte Licht. Störende Hornhautreflexe werden üblicherweise durch eine Teilung der Pupille des Auges verhindert. Dazu bildet die Ophthalmoskoplinse einen Beleuchtungsring in die Augenpupille ab. Die an der Hornhaut reflektierten Strahlen der Beleuchtung verfehlen die Apertur der Beobachtung. Nur das Areal innerhalb des Beleuchtungsringes wird für die Beobachtung verwendet.
Zur Unterdrückung der Reflexe von der Ophthalmoskoplinse sind es im wesentlichen zwei Konzepte bekannt.
In der DE-OS 35 19 442 ist ein optisches System beschrieben, bei welchem Lichtanteile, die über die Reflexion an der Ophthalmoskoplinse bzw. der Hornhaut in die Beobachtungsapertur gelangen könnten, mittels an geeigneter Stelle im Strahlengang angeordneten "Schwarzpunktplatten", welche in definierter Art und Weise mit lichtabsorbierenden Schichten belegt sind, ausgeblendet. Für diese Art der Reflexunterdrückung hat sich die Bezeichnung "Antireflexpunkt-Objektiv" eingebürgert.
Ein Nachteil dieses Konzeptes ist die Nähe des Antireflexpunktes zur Leuchtfeldblende. Die Absorption einzelner Lichtanteile kann als ungleichmäßige Ausleuchtung des Augenhintergrundes sichtbar werden, es treten ringförmige Schatten auf, welche den Bildeindruck verschlechtern und damit die Auswertung durch den Augenarzt behindern.
Ein andere Lösung ist in US 4,730,910 beschrieben. Dabei wird auf die Ausblendung bestimmter Lichtanteile innerhalb der Beleuchtungsoptik verzichtet. An Stelle der Ophthalmoskoplinse wird ein mehrlinsiges Objektiv verwendet, dessen Linsen so gegeneinander verkippt sind, dass die direkten Reflexe an den Glas-Luft-Flächen nicht in die Apertur der Beobachtung gelangen. Dazu liegen die optischen Achsen der Linsen gemeinsam mit der optischen Achse des Beobachtungsstrahlenganges in einer Ebene. Diese Lösung erfordert einen erheblichen Aufwand für die mechanischen Fassungen und weist erhebliche Probleme bei der Korrektion der Abbildungsfehler auf. US 4,415,239 zeigt eine gleiche lösung.
Ein solches System weist einen deutlichen Unterschied der Abbildungsmaßstäbe im Pupillenabbildung (Beleuchtung) sind anamorphotische Effekte zu beobachten. Die Bilder werden verzerrt, es liegt keine ähnliche Abbildung vor. Weiterhin zeigt das System Koma und Astigmatismus bei Abbildung des Achspunktes, die Abbildungsfehler im Feld sind nicht rotationssymmetrisch.

Die Erfindung stellt sich der Aufgabe, die Nachteile des Standes der Technik zu überwinden und ein optisches System für eine Funduskamera anzugeben, welches Reflexe an den optischen Flächen wirksam aus dem Abbildungsstrahlengang ausblendet und weitgehende Abbildungstrene zu gewährleisten.

Diese Aufgabe wird durch ein optisches System gemäß dem Hauptanspruch gelöst, vorteilhafte Weiterbildungen sind in den Unteransprüchen beschrieben.

Die Verkippung der Linsen in zwei, senkrecht aufeinander stehenden Ebenen bewirkt die Angleichung der Abbildungsmaßstäbe für zwei senkrecht aufeinander stehende Schnitte. Die Bilder werden weniger verzerrt und die Abbildung gewinnt an Ähnlichkeit. Darüber hinaus kann so der Achsastigmatismus korrigiert werden und die Rotationssymmetrie der Fehler im Feld annähernd wieder hergestellt werden.

Eine, bevorzugte Weiterentwicklung der Erfindung besteht aus vier sammelnden Linsen, welche in zweier Linsenpaare aufgeteilt sind. Die Linsen des ersten Linsenpaares werden in einer ersten Ebene verkippt. Die Linsen des zweiten Linsenpaares werden in einer zweiten Ebene verkippt, wobei diese beiden Ebenen senkrecht zueinander stehen. Es ist besonders vorteilhaft, wenn die Kippwinkel und Verschiebungen der Linsen so gewählt sind, dass der Bereich in der Nähe der optischen Achsen der Linsen nicht vom beleuchtenden Bündel durchdrungen wird.

Mit diesem System ist es möglich, die Abbildungsmaßstäbe für zwei senkrecht aufeinander stehende Schnitte sehr gut anzugleichen.

Die Erfindung wird im weiteren anhand der Figuren näher erläutert.
Es zeigen
Fig. 1 eine Seitenansicht,
Fig. 2 eine Draufsicht des erfindungsgemäßen optischen Systems,
Fig. 3 eine schematische Darstellung der Restverzeichnungen

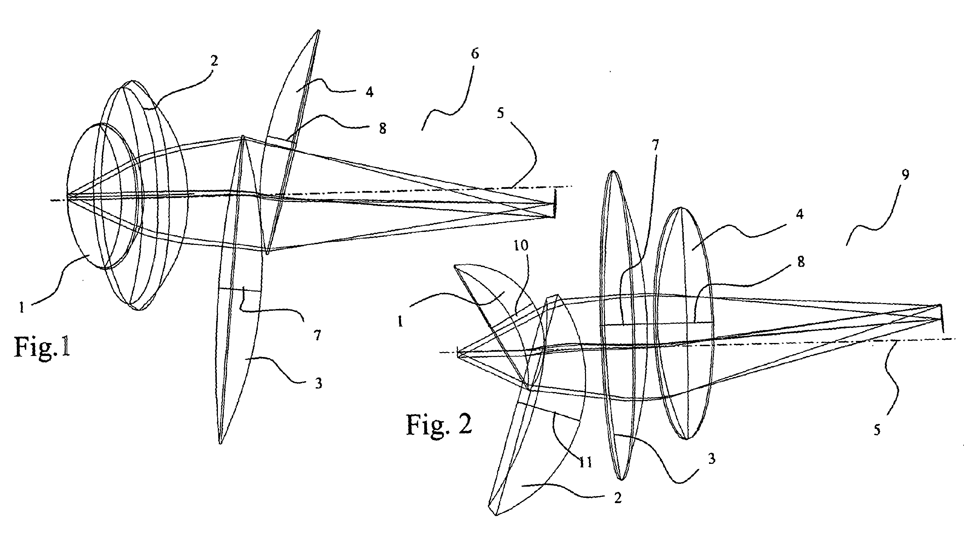

In Fig. 1 ist das erfindungsgemäße optische System schematisch in Seitenansicht dargestellt, wobei zur Vereinfachung auf die Darstellung der nicht erfindungswesentlichen Teile wie Patientenauge, Leuchtquellen, Fotoeinrichtung bzw. Beobachtungssystem verzichtet wurde.
Vier Linsen 1, 2, 3, 4 sind entlang der optischen Achse 5, welche den Beleuchtungs- und Abbildungsstrahlengang der Funduskamera definiert, derart angeordnet, dass die Linsen 1 und 2 gegenüber der Zeichenebene 6 von Fig. 1 verkippt sind, während die Linsen 3 und 4 innerhalb der Zeichenebene 6 gegen die optische Achse 5 verkippt sind, so dass deren optische Achsen 7 und 8 ebenfalls in der Zeichenebene 6 liegen.
Fig. 2 zeigt eine Draufsicht auf das optische System, die Zeichenebene 9 steht also senkrecht auf der Zeichenebene 6 aus Fig. 1. Die Linsen 1 und 2 sind innerhalb der Zeichenebene 9 so verkippt, dass deren optische Achsen 10 und 11 in der Zeichenebene 9 liegen. Die Linsen 3 und 4 sind damit gegenüber der Zeichenebene 9 verkippt.
Die Linsen sind dabei so angeordnet, dass das Beleuchtungsbündel des Abbildungs- und Beleuchtungsstrahlenganges mit der optische Achse 5 keine der Linsen 1, 2, 3, 4 in deren zentralem Bereich durchdringt, das heißt, die optischen Achsen 10, 11, 7, 8 der Linsen 1, 2, 3, 4 liegen außerhalb der in Fig. 1 und 2 schematisch dargestellten Bündelstrahlen.
In der folgenden Tabelle sind die optischen Daten einer bevorzugten Realisierung der Erfindung aufgeführt. Dabei stehen die Bezeichnungen mit dem Suffix v für die Vorderseiten und mit dem Suffix h für die Hinterseiten der Linsen 1, 2, 3, 4.

| *Nr.* | *Krümmungsradius* | *Abstand zur nächsten* | *Medium nach der* |
|---|---|---|---|
| | *[mm]* | *Fläche [mm]* | *Fläche* |
| *Obj.* | unendlich | 39,42477 | Luft |
| *1v* | -76,12524 | 16,63675 | LaK8 |
| *1h* | -50,66730 | -2,16757 | Luft |
| *2v* | -197,58358 | 29,93724 | LaK8 |
| *2h* | -89,73436 | 7,92140 | Luft |
| *3v* | 580,99844 | 26,79594 | LaK8 |
| *3h* | -260,25600 | -9,88489 | Luft |
| *4v* | 169,89283 | 20,11881 | LaK8 |
| *4h* | -1636,24830 | 32,91953 | Luft |
| | unendlich | 145,13560 | Luft |

Die folgendende Tabelle enthält die zur Realisierung dieses Ausführungsbeispiels notwendigen Werte für die Verschiebungen und Verkippungen der Linsen 1, 2, 3, 4 gegenüber der optischen Achse 5.

| *Fl.- Nr.* | *Dezentrierungsart* | *Verschiebung [mm]* | | *Drehung [*°*]* | | *Drehpunkt-Ablage in z-Richtung [mm]* |
|---|---|---|---|---|---|---|
| | | *x-Richtung* | *y-Richtung* | *um x-Achse* | *um y-Achse* | |
| *1v* | *1. Verschiebung, 2. Drehung* | *10,1400* | *1,0882* | *0,000* | *29,760* | *-23,8578* |
| *1h* | *1. Verschiebung, 2. Drehung* | *10,1400* | *1,0882* | *0,000* | *29,760* | *-40,4946* |
| *2v* | *1. Verschiebung, 2. Drehung* | *-6,5758* | *1,0882* | *0,000* | *-18,959* | *-93,6604* |
| *2h* | *1. Verschiebung, 2. Drehung* | *-6,5758* | *1,0882* | *0,000* | *-18,959* | *-123,5976* |
| *3v* | *1. Verschiebung, 2. Drehung* | *15,5328* | *-34,7519* | *5,988* | *0,000* | *-202,8670* |
| *3h* | *1. Verschiebung, 2. Drehung* | *15,5328* | *-34,7519* | *5,988* | *0,000* | *-229,6629* |
| *4v* | *1. Verschiebung, 2. Drehung* | *15,5328* | *-82,9383* | *14,841* | *0,000* | *465,0016* |
| *4h* | *1. Verschiebung, 2. Drehung* | *15,5328* | *-82,9383* | *14,841* | *0,000* | *444,8823* |
| | *1. Verschiebung, 2. Drehung, gedrehte Koordinaten gelten auch für folgende Flächen* | *3,7576* | *-5,0827* | *2,447* | *4,089* | *0,0000* |

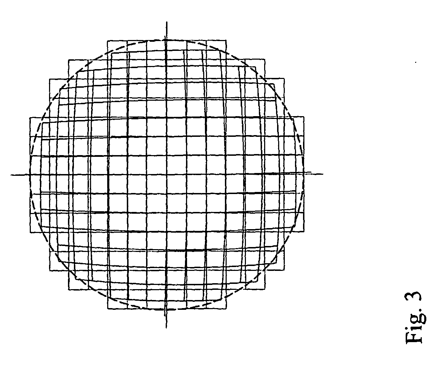

Die sich mit diesem System ergebenden Verzeichnungen sind in Fig. 3 dargestellt. Es zeigt sich, dass die Abbildungsmaßstäbe in x- und y-Richtung weitgehend übereinstimmen, das Vergleichsgitter (gerade Linien) hat in x- und y-Richtung die gleiche Ausdehnung. Damit sind die Anforderungen an die Ähnlichkeit der Abbildung sehr gut gewährleistet.
Das erfindungsgemäße optische System zeichnet sich weiterhin dadurch aus, dass die Fehler am Feldrand weitgehend rotationssymmetrisch ausgeprägt sind. Dadurch wird es möglich, diese Fehler durch ein rotationssymmetrisches Folgesystem zu kompensieren.

Die folgende Tabelle stellt die wesentlichen Daten eines optischen Systems nach dem Stand der Technik (US 4,730,910 = System A) und nach der Erfindung (System B) gegenüber.

| *Pupillenabbildung* | | System A | System B |
|---|---|---|---|
| Systembaulänge [mm] | | 60 | 104 |
| Brennweite f [mm] | | 31,5 | 57,3 |
| Pupillenabbildungsmaßstab | β*ₚₓ* | -2,19 | -2,5 |
| | β*_{py}* | -2,46 | -2,5 |
| Abbildungslänge *l'ₚ* [mm] | | 171,6 | 304 |
| Arbeitsabstand [mm] | | 24,2 | 38,1 |
| Feldwinkel Beleuchtung [°] | | 23,5 | 26 |
| mittl. Spotradius (Achse) [mm] | | 0,9974 | 0,2349 |
| mittl. Spotradius (Feld) [mm] | | 1,2532 | 0,4334 |
| Petzvalwölbung *rₚ* [mm] | | -71,9424 | -93,4579 |
| Verzeichnung | | 2,64% | 0,74% |
| Farblängsfehler *sₑ-s_{C'}* [mm] | | -3,13 | -5,76 |
| Farblängsfehler *sₑ-s_{F'}* [mm] | | 3,08 | 5,66 |

| *Objektabbildung* | | | |
|---|---|---|---|
| Feldwinkel Beobachtung [°] | | 22,5 | 25 |
| mittl. Spotradius (Achse) [mm] | | 0,0119 | 0,0020 |
| mittl. Spotradius (Feld) [mm] | | 0,0575 | 0,0158 |
| Verzeichnung | | -13,01% | -9,43% |
| Farblängsfehler *sₑ-s_{C'}* [µm] | | -17 | -7,6 |
| Farblängsfehler *sₑ-s_{F'}* [µm] | | 18 | 8,9 |

In allen für die Abbildungsqualität relevanten Parametern wird eine bedeutende Verbesserung gegenüber dem Stand der Technik erreicht.
Bei der Realisierung der Erfindung bietet es sich an, die in Fig. 1 und 2 dargestellten Linsen durch entsprechende Linsensegmente zu ersetzen, welche mindestens den Bereich der Durchdringung des Beleuchtungs- und Abbildungsstrahlenganges umfassen.
Die Erfindung ist nicht an das dargestellte Ausführungsbeispiel gebunden, in bestimmten Fällen kann es auch günstig sein, wenn die beiden Verkippungsebenen nicht senkrecht aufeinander stehen.
Zur Verbesserung der optischen Abbildungseigenschaften kann es vorteilhaft sein, mindestens eine der Linsen mit einer asphärischen Fläche zu versehen. Eine andere Realisierung der Erfindung ist durch den Einsatz diffraktiver optischer Elemente an statt einer oder mehrerer der Linsen möglich.
Das Prinzip der Erfindung ist auch in anderen Gebieten anwendbar, bei denen die Aufgabe besteht Reflexe an den optischen Flächen zu unterdrücken.

## Patentansprüche

1. Optisches System für eine Funduskamera, welche einen im wesentlichen koaxialen Beleuchtungs- und Abbildungsstrahlengang mit einer optischen Achse (5) aufweist, bestehend aus einem Linsensystem von mindestens vier Linsen (1, 2, 3, 4), wobei mindestens zwei Linsen (3, 4) bezüglich ihrer optischen Achsen (7, 8) gegen den Beleuchtungs- und Abbildungsstrahlengang (5) verkippt sind, wobei die optischen Achsen (7, 8) der Linsen (3, 4) und die optische Achse des Beleuchtungs- und Abbildungsstrahlenganges (5) in einer Ebene (6) liegen,
**gekennzeichnet dadurch, dass** mindestens zwei weitere Linsen (1, 2) bezüglich ihrer optischen Achsen (10, 11) gegen den Beleuchtungs- und Abbildungsstrahlengang verkippt sind,
dass die optischen Achsen (10, 11) der zwei weiteren Linsen (1, 2) und die optische Achse (5) des Beleuchtungs- und Abbildungsstrahlengangs in einer zweiten Ebene (9) liegen, die die erste Ebene (6) im wesentlichen entlang der optischen Achse (5) des Beleuchtungs- und Abbildungsstrahlenganges schneidet,
dass die erste und die zweite Ebene (6, 9) im wesentlichen senkrecht zu einander stehen
und dass die optische Achse des Beleuchtungs- und Abbildungsstrahlengangs (5) die Linsen (1, 2, 3, 4) außerhalb deren optischer Achsen (7, 8, 10, 11) durchdringt.

2. Optisches System nach Anspruch 1, **gekennzeichnet dadurch, dass** die optischen Achsen der Linsen außerhalb des Strahlenbündels des Beleuchtungs- und Abbildungsstrahlengangs angeordnet sind.

3. Optisches System nach Anspruch 1 oder 2, **gekennzeichnet dadurch, dass** die Linsen aus Linsensegmenten bestehen.

4. Optisches System nach Anspruch 1, 2 oder 3, **gekennzeichnet dadurch, dass** mindestens eine der Linsen eine asphärische Fläche aufweist.

5. Optisches System nach Anspruch 1, 2, 3 oder 4, **gekennzeichnet dadurch, dass** mindestens eine Linse durch ein diffraktives optisches Element ersetzt wird.

## Claims

1. Optical system for a fundus camera which has a substantially coaxial illumination and imaging beam path having an optical axis (3), the system consisting of a lens system of at least four lenses (1, 2, 3, 4), wherein at least two lenses (3, 4) are, in relation to their optical axes (7, 8), tilted towards the illumination and imaging beam path (5), wherein the optical axes (7, 8) of the lenses (3, 4) and the optical axis of the illumination and imaging beam path (5) are located in one plane (6),
**characterised in that** at least two further lenses (1,2) are, in relation to their optical axes (10, 11), tilted towards the illumination and imaging beam path,
**in that** the optical axes (10, 11) of the two further lenses (1, 2) and the optical axis (5) of the illumination and imaging beam path are located in a second plane (9) which intersects the first plane (6) substantially along the optical axis (5) of the illumination and imaging beam path,
**in that** the first and second planes (6, 9) are substantially perpendicular to each other, and **in that** the optical axis of the illumination and imaging bean path (5) passes through the lenses (1, 2, 3, 4) outside their optical axes (7, 8, 10, 11).

2. Optical system as claimed in Claim 1, **characterised in that** the optical axes of the lenses are arranged to be outside the beam bundle of the illumination and imaging beam path.

3. Optical system as claimed in Claim 1 or 2, **characterised in that** the lenses consist of lens segments.

4. Optical system as claimed in Claim 1, 2, or 3, **characterised in that** at least one of the lenses has a non-spherical surface.

5. Optical system as claimed in Claim 1, 2, 3 or 4, **characterised in that** at least one lens is replaced by a diffractive, optical element.

## Revendications

1. Système optique pour une caméra à fond d'oeil qui présente un chemin optique d'illumination et de représentation sensiblement coaxial avec un axe optique (5), constitué d'un système de lentilles formé par au moins quatre lentilles (1, 2, 3, 4), où au moins deux lentilles (3, 4) sont basculées relativement à leurs axes optiques (7, 8) contre le chemin optique d'illumination et de représentation (5), où les axes optiques (7, 8) des lentilles (3, 4) et l'axe optique du chemin optique d'illumination et de représentation (5) se situent dans un plan (6),
**caractérisé en ce qu'**au moins deux autres lentilles (1, 2) sont basculées relativement à leurs axes optiques (10, 11) contre le chemin optique d'illumination et de représentation,
**en ce que** les axes optiques (10, 11) des deux autres lentilles (1, 2) et l'axe optique (5) du chemin optique d'illumination et de représentation se situent dans un second plan (9) qui coupe le premier plan (6) sensiblement le long de l'axe optique (5) du chemin optique d'illumination et de représentation,
**en ce que** le premier et le second plan (6, 9) s'étendent sensiblement perpendiculairement l'un à l'autre,
et **en ce que** l'axe optique du chemin optique d'illumination et de représentation (5) traverse les lentilles (1, 2, 3, 4) à l'extérieur de leurs axes optiques (7, 8, 10, 11).

2. Système optique selon la revendication 1, **caractérisé en ce que** les axes optiques des lentilles sont disposés à l'extérieur du faisceau de rayons du chemin optique d'illumination et de représentation.

3. Système optique selon la revendication 1 ou 2, **caractérisé en ce que** les lentilles sont constituées de segments de lentille.

4. Système optique selon la revendication 1, 2 ou 3, **caractérisé en ce qu'**au moins une des lentilles présente une face non-sphérique.

5. Système optique selon la revendication 1, 2, 3 ou 4, **caractérisé en ce qu'**au moins une lentille est remplacée par un élément optique de diffraction.
